# EUROPEAN PATENT SPECIFICATION

(11) **EP 2 766 727 B1**
(45) Date of publication and mention of the grant of the patent: **07.10.2015**
(21) Application number: 12787472.5
(22) Date of filing: 30.08.2012
(51) Int. Cl.: G01N 21/84, G01N 33/52, G01N 33/53, G01N 33/558

(54) **ASSAY DEVICE COMPRISING AN OPTICAL FILTER AND A METHOD OF ASSAYING**
ASSAYVORRICHTUNG MIT EINEM OPTISCHEN FILTER UND ASSAYVERFAHREN
DISPOSITIF DE DOSAGE COMPRENANT UN FILTRE OPTIQUE ET PROCÉDÉ DE DOSAGE

(30) Priority: 30.08.2011 US 201113221381; 24.11.2011 GB 201120356
(43) Date of publication of application: 20.08.2014
(73) Proprietor: Supernova Diagnostics, Inc., Germantown, Maryland 20876 (US)
(72) Inventor: CAMPBELL, Neil, J., Damascus, MD 20872 (US); MORAVICK, Keith, Edward, Mountain View, CA 95043 (US); RICHARDSON, Bruce, J., Los Gatos, CA 95032 (US)
(74) Representative: Beresford, Keith Denis Lewis
(86) International application number: PCT/GB2012/000691
(87) International publication number: WO 2013/030524

(56) References cited:
- CN-A- 101 726 596
- US-A1- 2005 243 321
- US-A1- 2007 231 922
- US-A1- 2008 199 851
- US-A1- 2009 211 345

## Description

### BACKGROUND OF THE INVENTION

This invention relates generally to devices for bio-assay, pathogen, or molecular analysis, and to systems and methods for such analysis. The invention may be employed for bio-assays such as sandwich or competitive assays, e.g. immunoassays, for example fluorescence immunoassays (FIA).

Optical systems may be used to determine the presence, concentration or quantity of a target material or analyte in a test sample, quantitatively, semi-quantitatively or qualitatively. However, while such systems may be used successfully, they suffer from a number of disadvantages. For example, they may require the use of relatively expensive equipment in order to perform the assay which may not be appropriate for some determinations or may not be available for some tests. For example, in some cases a qualitative determination of the presence' of an analyte may be sufficient whereas in other cases a quantitative determination may be necessary. Furthermore, such tests may be of limited performance leading to tests having to be repeated. In addition, there is a general desire to reduce the cost of the equipment used for the assay, and to reduce the time taken to perform the test. In other cases, it may not be desired to perform such an assay under laboratory conditions but instead it may be necessary to perform the test in the field with the minimum of equipment.

US2005/243321 A1 discloses a system that employs transmission-based detection techniques to determine the presence or concentration of an analyte within a test sample is provided. Specifically, the optical detection system contains a chromatographic-based assay device that is positioned in the electromagnetic radiation path defined between an illumination source and detector. To enhance the sensitivity and signal-to-noise ratio of the system without significantly increasing costs, the distance between the illumination source and/or detector and the assay device is minimized. The illumination source and/or detector may also be positioned directly adjacent to the assay device. In addition, the system may be selectively controlled to reduce reliance on external optical components, such as optical filters or diffusers.

### SUMMARY OF THE INVENTION

According to one embodiment, the present invention provides a stand-alone assay device according to independent claim 1.

The device may be employed for performing lateral flow tests or other forms of test such as flow-through devices and tests and microfluidic tests, indeed for any test that may be used for competitive or sandwich assays.

This form of device may be employed in a number of ways. For example, it may be used together with an illumination and reading device in order to detect very small quantities of the analyte, or to perform quantitative tests on the analyte. However, in other circumstances the device may be a free standing or stand-alone device which may be employed without a reader in order to perform a less sensitive or qualitative test simply to determine the presence or absence of an analyte.

According to another embodiment, the invention provides an arrangement for performing an assay according to claim 8.

According to yet another embodiment, the invention provides a method of performing a test on an analyte according to independent claim 9.

Normally, the assay device will include a label zone where a quantity of the label matenal is located, the label zone preferably being located on the pathway between the application zone and the capture zone so that, on application of the analyte the analyte will contact the label material, and the analyte and label material will together pass along the pathway to the capture zone. However, in the broadest aspect of the invention, it is not necessary for the label material to be located on the pathway of the device when the assay device is supplied, and it is possible for the label material to be supplied separately and to be applied to the device along with the analyte. Alternatively, the label material may be mixed with the analyte beforehand, i.e., before applying the analyte to the device.

### BRIEF DESCRIPTION OF THE DRAWINGS

The invention will now be described by way of example only with reference to the drawings, in which:
Figure 1 is a schematic perspective view of a test device according to the present invention, and a reader;
Figure 2 is a more detailed view of the test device of Figure 1;
Figure 3 is an exploded view of the test device shown in Figure 2 showing the various components thereof;
Figure 4 is a graphical representation of emission and absorption spectra of one form of label material that may be employed in the device;
Figure 5 is a view of the device with the top surface removed to show the inner details thereof;
Figure 6 is a schematic view of the device and reader;
Figure 7 is a view of the device during a test according to the invention;
Figure 8 shows the device at one point during the test; and
Figure 9 shows the device at another point during the test.

### DETAILED DESCRIPTION OF THE INVENTION

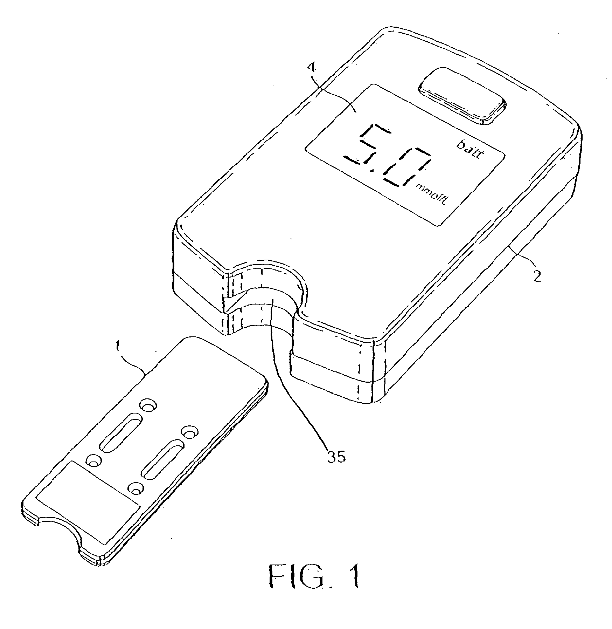

Figure 1 shows schematically an assay device or test strip 1 and a reader 2 for enabling a quantitative or qualitative test to be performed on an analyte. In one embodiment of the invention, a quantity of an analyte may be applied to one part of the device so that it can flow along a pathway in the device toward the opposite end thereof, and the device can be inserted into the reader 2 in order to obtain an output indicative of the quantity of the analyte on an output display 4 of the reader.

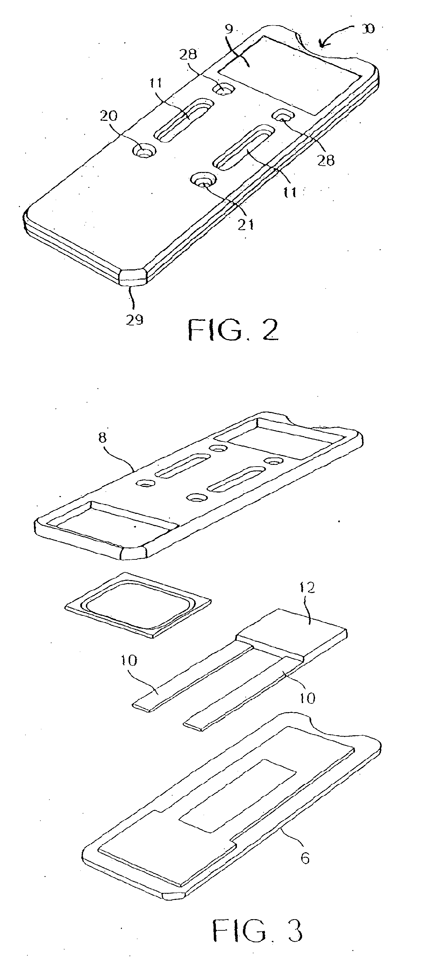

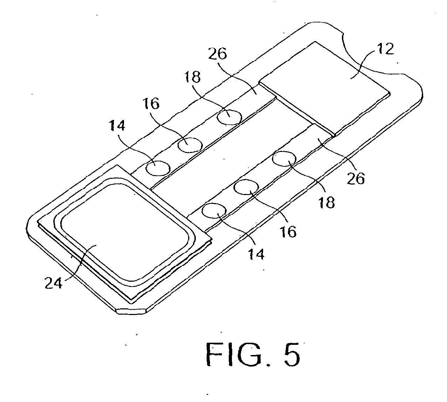

The form of the assay device is shown in more detail in Figures 2 and 3. As shown in Figure 3, the device comprises a generally transparent bottom cover 6 and a generally transparent top cover 8 which is located over the bottom cover and bonded thereto. The top cover 8 may include an area 9 on which a physical label may be affixed for information. The top and bottom covers may be transparent over their entire surface which enables them to be made from a single piece of plastics material. However, this is not essential, and it is necessary only for the top and bottom covers to be transparent in the region of pathways 10 along which the analyte will be caused to travel. The top and bottom *covers* enclose a plurality of pathways 10, two in this case, one for detection of the analyte and a control pathway, although other numbers may be used if, for example, a number of different analytes are to be detected using the same assay device. The pathways are formed from a porous material such as nitrocellulose. The pathways 10 extend from a sample dispensing zone or application zone 14 (see Figure 5) at one end region of the device where the analyte may be dispensed during the test to the opposite end region so that the analyte will travel along the pathway during the test. In this form of assay device, the thickness of the transparent top cover 8 may be reduced along a reduced thickness region 11 extending along part of the pathway 10 in order to allow the pathway to be viewed more easily through the transparent top cover 8. A wash collection reservoir 12 may be provided in the opposite end region of the device so that wash may be applied to the pathways to cause the analyte to flow along the pathway to the wash collection reservoir.

A quantity of a label, which as used herein includes any label precursor, may be provided at a label and primary receptor zone 16 on one or both of the pathways at a position between the sample dispensing zone and the waste zone or wash collection reservoir so that, as the analyte travels along the pathway, the label will be taken up by the analyte and will travel along the pathway together with the analyte. A specific bond may be formed between the label and the analyte, for example a bond between an antibody pair, an antigen pair, a DNA bond or any other bond. In one example, where the analyte is avidin, streptavidin or neutravidin, the label material may be biotinylated so that it will form a biotin-avidin bond with the analyte. The presence of the analyte may thus be detected by subsequently observing the presence of the label. The label is described in greater detail below.

Downstream of the label and primary receptor zone 16 along the pathway is a capture zone 18 (see Figure 5) where a quantity of capture material is located. The capture material is bound to the pathway and will bind to any of the analyte that passes along the pathway so that the analyte will be retained in one position on the pathway 10 corresponding to the capture material. In the case of a device that is used to detect avidin, streptavidin or neutravidin mentioned above, the capture material may also be biotinylated so that the analyte will be bound to the pathway by a biotin-avidin bond.

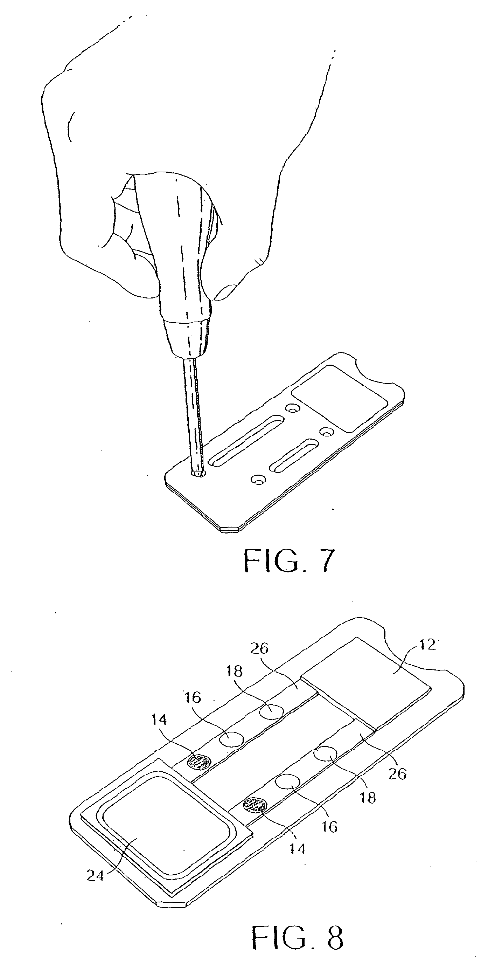

During the assay, a quantity of the analyte is dispensed into a dispensing port 20 (see Figure 2) that is located over one of the pathways above the application zone 14. The analyte may be dispensed by means of a pipette, dropper or other appropriate device as shown in Figure 7 in order to dispense a defined quantity of the analyte. A quantity of a control analyte may be applied to a dispensing port 21 located over the application zone of the other pathway 10. The control analyte may be one that exhibits a known strong bond with the label material and capture material, and a control label may be present (which may be the same or different to the label employed in the other pathway) in order to exhibit a strong, defined signal when the device is illuminated in order to enable the operator to confirm that analyte has been dispensed during the assay.

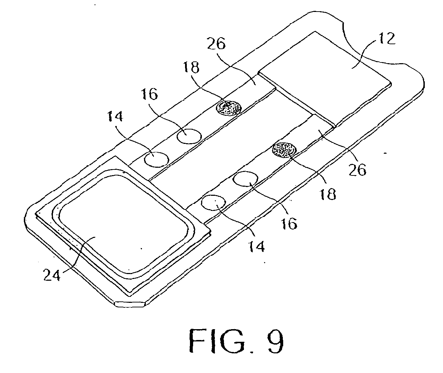

In the case of a quantitative analysis, it is possible to use the signal generated from the control label as a reference against which the signal from the analyte/label signal may be compared. For example the control analyte/control label bond and control analyte/control capture material bond may be based on a biotin-avidin bond or a similar bond as mentioned above. Initially the analyte will wet the pathways located under the dispensing ports 20 and 21 as shown by the hatched regions in Figure 8. The analyte will travel along the pathway by capillary action taking the label from the label zone 16 of Figure 5 with it. At the capture zone 18, the analyte and label will bond to the pathway as shown by the hatched region in Figure 9. Downstream of the capture zone 18 on the pathway or each of the pathways 10 is a process control zone 26 which is located below a process control window 28 in the top surface of the device. After application of analyte it will travel along the pathway by capillary action, and will hydrate the pathway causing a colour change. The pathway can be viewed through the process control windows 28 in order to observe the colour change and ensure that the analyte and label have travelled along the pathway to and beyond the capture zone 18.

A wash may then be applied to cause the analyte, control analyte and label to flow along the pathways toward the wash collection reservoir 12. The wash may be applied in any number of ways. For example it may be applied to the sample dispensing port, or an alternative port, by means of a pipette, dropper or other device that will dispense a quantity of wash into the dispensing ports 20 and 21. Alternatively, a wash dispensing reservoir 24 may be provided in the device at the end of the device opposite the wash collection reservoir, or on a side of the device. The dispensing reservoir may be in the form of a bladder, blister pack or sachet which may be punctured in order to cause the wash to flow along the pathways. In another form of arrangement where a reader is employed as described below, the reader may be designed to receive the assay device and be provided with a ridge or protuberance that will apply a stress to the wash dispensing reservoir and rupture it when the device is inserted into the reader so that the wash is applied automatically on insertion of the assay device into the reader.

Once the wash has been applied, substantially no material should be present on the pathway other than the analyte bound to the capture zone and the label that is bound to the analyte.

With many forms of capture and label arrangement the assay device may be ready to be illuminated and read as soon as the device is washed. However, in some cases a further processing step may be necessary in order to activate the label (which may be referred to herein at times as a label precursor). For example, where some forms of fluorescent label are used as described below, and in particular where fluorescein diacetate (FDA) is used, it may be necessary to hydrolyse the precursor and/or to heat it in order to release the label. This operation may be performed at any appropriate time. For example, if an acid or base hydrolysis step is necessary, the appropriate acid or base may be applied either before or after application of the analyte, for example along with the wash step. In this case, the wash in the wash reservoir may have a pH that will cause the hydrolysis so that the activation step occurs automatically with the washing step.

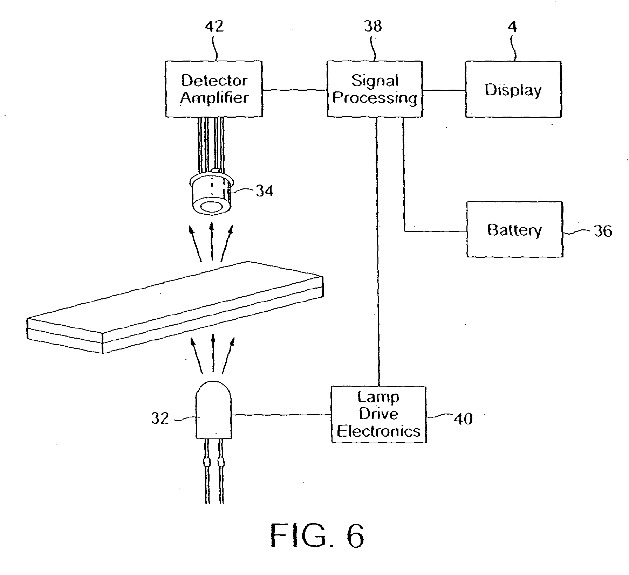

The assay device is now ready to be illuminated in order to detect the presence of analyte. This may be achieved by means of a reader as shown in Figures 1 and 6. The reader comprises a housing having an orifice or slot 35 therein for receiving the assay device 1, a light source 32 for example an LED or an incandescent lamp, located on one side of the slot for receiving the assay device, and an optical sensor for example a PIN diode or an avalanche photodiode located on the opposite side of the slot so that light from the light source will pass through the device. The light source may be powered by a standard battery 36 or other power source such as a transformer. The reader includes a conventional signal processor 38 for controlling light source drive electronics 40, a detector amplifier circuit 42 and a display circuit for the display 4.

The assay device is preferably provided with a polarizing profile in order to ensure that the device cannot be inserted into the reader upside down or back to front. As will be appreciated, inserting the device upside down will cause any excitation wavelengths to be filtered out by the longer wavelength filter on the upper surface of the device. Such a polarizing profile may include a cut corner 29 which will cooperate with a corresponding corner in the reader. Also, a part-circular cut out 30 may be included in one end of the device to ensure which end of the device is inserted. Many other forms of polarizing profile may be employed.

In some cases, the assay device may be illuminated as soon as the pathways have been washed, but in other cases it may be necessary to wait for a period of time before illumination, in which case a timer may be provided in the reader in order to ensure that the device is illuminated and read at the appropriate time. For example, in the case of base hydrolysed FDA, it may be advantageous to wait for a period of from 100 to 500 seconds after hydrolysis before illumination.

Although the term "light" has been used herein, it will be appreciated that this is because the device is intended for reading visually. Any electromagnetic radiation may in principle be used to read the device, and the light will not necessarily be visible light, although this is preferred. The light may have components in the infrared or ultraviolet spectrum and may even have a spectrum in which the radiation is predominantly in wavelength ranges outside the visible wavelength range. However, as explained below, the light is preferably in the visible range.

The label material located in the device may be one that will emit or modify light, at least when activated, so that the light emitted from the device due to the presence of the analyte will differ from the illuminating light.

One surface of the device that receives the light only after it has passed through the label material, in this embodiment the top cover 8, is formed from a material that provides a first optical filter that will allow transmission of the light emitted or modified by the label material and will block light of at least one other wavelength range. This has the advantage that the effect of the label material is enhanced by removing at least some of the background light that is not affected by the label material.

The other surface of the device, that is to say the surface that will be illuminated by the light source during the reading stage, is also formed from a material that forms a second optical filter that has a different optical transmission characteristic from that of the first optical filter.

The second optical filter will allow transmission of light of a shorter wavelength range than that of the first optical filter, for example the second optical filter may be a blue filter while the first optical filter may be a green filter. Especially the filters will be such that together they will block light in substantially the entire visible light range. In other words, in the preferred form of device, the long wavelength cut off of the second filter will be substantially the same as, or in the region of the short wavelength cut off of the first filter, so that the combination of the two filters will block substantially all visible light.

The filters provided in the top and bottom of the assay device may be formed from any appropriate material, for example glass, plastics materials, thin film materials or they may be holographic filters or interference filters. In an interference filter, a dielectric coating is deposited in layers to allow only the desired wavelengths to pass while light of other wavelengths is reflected. However, in view of the fact that the filters are provided on the assay device which will be a consumable item, the materials should be relatively inexpensive and so plastics filters are preferred.

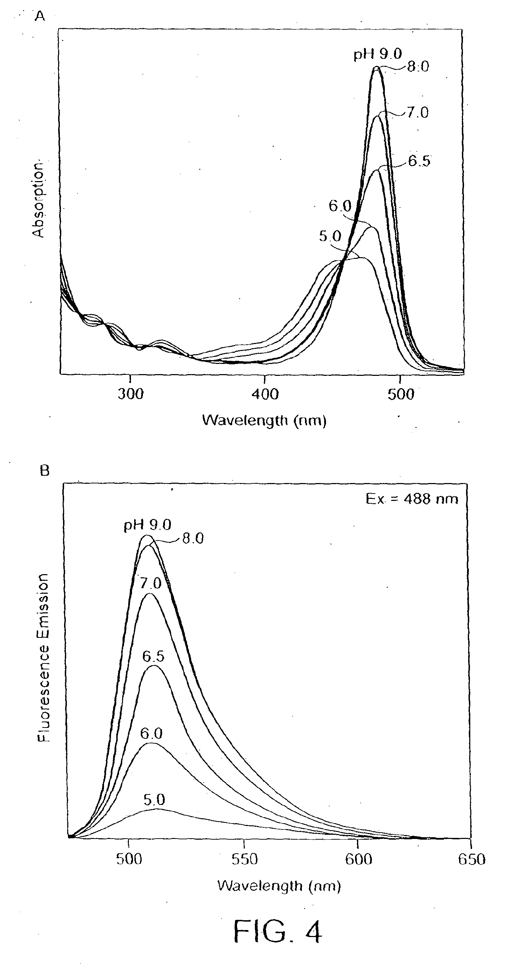

If the label material is one such as a fluorescent or phosphorescent material which exhibits a Stokes shift between its absorption spectrum and emission spectrum, it is possible for the filters formed by both surfaces of the device to block substantially all light but allow fluorescence or phosphorescence caused by the label to be detected. For example, Figure 4 shows the absorption spectrum of fluorescein (graph A) which may be employed as a label material with a maximum at 492nm and its emission spectrum (graph B) with a maximum at 517nm. As can be seen the use of a second optical filter having a long wavelength cut off in the region of 500nm and a second optical filter having a short wavelength cut off in the region of 500nm will allow substantially the entire fluorescence from fluorescein to be observed against a dark background.

The use of labels that can affect the wavelength of light such as fluorescent or phosphorescent materials together with optical filters on both sides of the assay device as described above (rather than the filters being associated with the reader) has the important advantage that it is possible, at least for a number of tests (for example many qualitative tests or where a high concentration of analyte is present) to dispense with the optical reader so that it is possible simply to hold the assay device up to a source of white light, for example the sun, and observe the presence or absence of any bands on the pathway caused by the fluorescent label.

According to the broadest aspect of the invention, any of a number of label materials may be employed in the device. These may include simple coloured dyes or pigments that will affect the absorption spectrum of the analyte, but they are preferably fluorescent, phosphorescent or chemiluminescent materials. Examples of materials that may be employed as labels are disclosed in U.S. patent No. 7,796,266. In addition, the term "label" as used herein can include precursors of a label where appropriate, so that some additional step or steps may be needed before the material functions as an optical label, for example acid or base hydrolysis may be required or the application of heat or both.

According to one preferred embodiment, the label material comprises lipid walled capsules, optionally having a polymer outer shell, containing a signal precursor. Such forms of label material are disclosed in international patent application No. WO 02/12888 A2.

For example, the capsules may be formed from the lipid DSPE-PEG2000 Amine and sodium dodecyl sulphate (SDS) and may contain fluorescein diacetate (FDA) as the signal precursor. These capsules may be activated by being placed in an activation solution having a pH of approximately 10.1 and then heating. The pH of the activation solution is chosen to be just below the pH value at which the FDA in this type of capsule will undergo rapid hydrolysis to fluorescein without additional heat.

Capsules such as those disclosed in international patent application No. WO 02/12888 A2 release very large amounts of fluorescent or phosphorescent material when activated. That is to say, the capsules contain many thousands of millions of molecules of the fluorescent or phosphorescent material, with the result that assays employing these capsules can be extremely sensitive since the intensity of the emitted light can be many orders of magnitude above that of other assays using non-encapsulated fluorescent or phosphorescent materials. Each capsule is potentially able to become bound to a single molecule of the target material in the analyte solution. Then, when the fluorescent or phosphorescent material is released from the bound capsule, many thousands of millions of signal generating molecules are released for each molecule of the target material. Hence, the assay is highly sensitive to even small quantities of target material.

For example, for the FDA-containing capsules, it is conjectured that it is this very high degree of fluorescence generated by the FDA-containing capsules when activated that enables the FDA-containing capsules to be employed in an assay device according to an embodiment of the present invention employing relatively cheap and low performance optical filters located on a consumable component such as the assay device. According to U.S. patent No. 7,796,266 referred to above, it is a large Stokes shift, for example from 100nm to 350nm, that minimizes the need for expensive, high precision filters in the optical detection in order to eliminate background interference. However fluorescein employed according to an embodiment of the present invention has a Stokes shift of only about 25 to 28nm.

In fact, the use of fluorescent or phosphorescent labels, and especially labels formed from the FDA-containing capsules referred to above, can have the effect that the assay device can exhibit an absorption spectrum of fluorescein when exposed to white or ambient light. Thus, according to yet another aspect, an embodiment of the method according to the invention includes the step of illuminating the assay device from one side with white light or ambient and viewing the device from the other side in order to detect absorption bands in the pathway caused by absorption of light by the fluorescent label material. In this method it is not necessary to use a reader with a dedicated light source *and* detector. Nor is it necessary to include any second optical filter on the side of the device that is illuminated although it may be advantageous to do so in order to reduce the intensity of light passing through the assay device that is not affected by the absorption by the fluorescent or phosphorescent material, and so increase the proportion of light that is absorbed by the fluorescent or phosphorescent material.

The ability of encapsulated signal generating molecules to generate detectable signals for extremely low concentrations of target material gives exceptional utility to the assay device of the present invention. When such encapsulated signal generating molecules are incorporated into or used with the assay device, point of care determinations can be made even though the user may not have access to a reader. Hence, the device can be used in remote locations, in field applications, or in any circumstances where access to a reader is not available.

## Claims

1. A stand-alone assay device (1) for enabling a test to be performed on an analyte, the assay device (1) having an application zone (14) for application of a quantity of the analyte, a waste zone (12), and a pathway (10) for allowing passage of the analyte from the application zone (14) to the waste zone (12);
the assay device (1) having a first cover (6) on a first side of the pathway (10) and a second cover (8) on a second side of the pathway (10) opposite the first side;
the first cover (6) providing a first optical filter for allowing transmission of light emitted or modified by a label material and for blocking light of at least one other wavelength range; and
the second cover (8) forming a second optical filter, the second optical filter having a different optical transmission characteristic from that of the first optical filter;
the assay device (1) including a quantity of the label material which will emit or modify light at least when activated and which will bind to the analyte, the pathway (10) also including a capture zone (18) located between the application zone (14) and the waste zone (12) that has a quantity of capture material which is bound to the pathway (10) and which will bind to analyte that passes along the pathway (10) so that the analyte will be bound to the pathway (10), the assay device (1) being adapted to be illuminated from said second side of the pathway by a light source (32) separate from the assay device (1) such that light emitted or modified by the label material located in the pathway (10) is detectable at the first side of the pathway.

2. An assay device (1) as claimed in claim 1, wherein each of the first and second covers (6,8) comprises a single piece of plastics material.

3. An assay device (1) as claimed in claim 1, wherein the first optical filter and the second optical filter together block substantially all light.

4. An assay device (1) as claimed in claim 1 wherein the second optical filter will allow transmission of light of shorter wavelength than light transmitted by the first optical filter.

5. An assay device (1) as claimed in claim 4 wherein the label material is a fluorescent or phosphorescent material or a precursor thereof, and the first optical filter will allow transmission of light only after modification by the label material.

6. An assay device (1) as claimed in claim 1 wherein the first optical filter will allow transmission of light of different wavelengths that correspond to light emitted or modified by different label materials.

7. An assay device (1) as claimed in claim 1 which includes a plurality of pathways (10) extending generally in parallel with one another from an application zone (14) to a waste zone (12), the pathways (10) containing different capture materials to enable a test to be performed on a plurality of different analytes.

8. An arrangement for performing an assay, which comprises an assay device (1) as claimed in claim 1 and a reader (2) for detecting an output from the assay device (1), said reader (2) comprising a body having an orifice (35) that is capable of receiving the assay device (1), a light source (32) for illuminating the assay device (1) from said second side thereof once it has been received in the reader (2), a terminal for receiving a power source (36) for the light source (32), an optical detector (34) located in the body for detecting light that has been emitted from the first side of the assay device, and a detector (38,42) for detecting the optical power of light detected by the optical detector (34).

9. A method of performing a test on an analyte, by means of a stand-alone assay device (1) having an application zone (14) for application of the analyte, a waste zone (12), and a pathway (10) for allowing passage of the analyte from the application zone (14) to the waste zone (12), the assay device (1) having a first cover (6) on a first side of the pathway (10) and a second cover (8) on a second side of the pathway (10) opposite the first side, the pathway (10) also including a capture zone (18) located between the application zone (14) and the waste zone (12) that has a quantity of capture material which is bound to the pathway (10) and which will bind to any analyte, which method comprises:
applying a quantity of the analyte to the application zone (14) and allowing the analyte to pass along the pathway (10) to and beyond a capture zone (18) where the pathway (10) includes a quantity of capture material which will bind the analyte to the pathway (10);
causing the analyte to contact, either before or after it has reached the capture zone (18), a quantity of a label material which will emit or modify light at least when activated and which will bind to the analyte so that the analyte and label material will be bound to the pathway (10) at the capture zone (18);
applying a quantity of a wash to the application zone (14) in order to cause excess analyte and label material to flow along the pathway (10) to the waste zone (12); and
illuminating the assay device (1) from the second side of the pathway and
detecting light that is emitted or modified by the label material from the first side of the pathway, said first cover (6) of the assay device (1) providing a first optical filter for allowing transmission of light emitted or modified by the label material and for blocking light of at least one other wavelength range so that detection of the light will indicate the presence or quantity of the analyte, and said second cover of the assay device (1) forming a second optical filter having a different optical transmission characteristic from that of the first optical filter.

10. A method as claimed in claim 9, wherein each of the first and second covers (6,8) comprises a single piece of plastics material.

11. A method as claimed in claim 9, wherein the first optical filter and the second optical filter together blocks substantially all light.

12. A method as claimed in claim 9 which comprises inserting the assay device (1) into a reader (2) for detecting an output from the assay device (1), the reader (2) comprising a body having an orifice (35) that is capable of receiving the assay device (1), a light source (32) for illuminating the assay device (1) from said second side thereof once it has been received in the reader (2), an optical detector (34) located in the body for detecting light that has been emitted from first side of the assay device (1), and an arrangement (38,42) for detecting the optical power of light detected by the optical detector (34).

13. A method as claimed in claim 9 which comprises illuminating the assay device (1) with white light from said second side thereof and observing from the first side thereof to determine the presence or quantity of analyte from the label material.

14. A method as claimed in claim 13 wherein the white light is ambient light.

15. A method as claimed in claim 13 or claim 14 wherein the label material comprises a fluorescent or phosphorescent material.

## Patentansprüche

1. Eigenständige Untersuchungsvorrichtung (1), die ermöglicht, dass ein Test eines Analyten durchgeführt wird, wobei die Untersuchungsvorrichtung (1) eine Aufbringungszone (14) für das Aufbringen einer Menge des Analyten, eine Abfallzone (12) und eine Bahn (10) hat, um den Durchhang des Analyten von der Aufbringungszone (14) zu der Abfallzone (12) zuzulassen,
wobei die Untersuchungsvorrichtung (1) eine erste Abdeckung (6) auf einer ersten Seite der Bahn (10) und eine zweite Abdeckung (8) auf einer zweiten Seite der Bahn (10) gegenüber der ersten Seite hat;
wobei die erste Abdeckung (6) ein erstes optisches Filter bereitstellt, um die Transmission von Licht zuzulassen, das von einem Kennzeichnungsmaterial emittiert oder modifiziert wird, und um Licht wenigstens eines anderen Wellenlängenbereichs zu sperren, und
wobei die zweite Abdeckung (8) ein zweites optisches Filter bildet, wobei das zweite optische Filter eine andere optische Transmissionscharakteristik als die des ersten optischen Filters hat;
wobei die Untersuchungsvorrichtung (1) eine Menge des Kennzeichnungsmaterials umfasst, die zumindest, wenn sie aktiviert wird, Licht emittieren oder modifizieren wird und die mit dem Analyten binden wird, wobei die Bahn (10) auch eine Einfangzone (18) umfasst, die zwischen der Aufbringungszone (14) und der Abfallzone (12) angeordnet ist, die eine Menge an Einfangmaterial hat, das an die Bahn (10) gebunden ist, und die sich an den Analyten binden wird, der entlang der Bahn (10) vorbei läuft, so dass der Analyt an die Bahn (10) gebunden wird, wobei die Untersuchungsvorrichtung (1) geeignet ist, von der zweiten Seite der Bahn durch eine von der Untersuchungsvorrichtung (1) getrennte Lichtquelle (32) beleuchtet zu werden, so dass Licht, das von dem Kennzeichnungsmaterial, das sich in der Bahn (10) befindet, emittiert oder modifiziert wird, an der ersten Seite der Bahn detektierbar ist.

2. Untersuchungsvorrichtung (1) nach Anspruch 1, wobei jede der ersten und zweiten Abdeckungen (6, 8) ein einziges Stück Kunststoffmaterial aufweist

3. Untersuchungsvorrichtung (1) nach Anspruch 1, wobei das erste optische Filter und das zweite optische Filter zusammen im Wesentlichen jegliches Licht sperren.

4. Untersuchungsvorrichtung (1) nach Anspruch 1, wobei das zweite optische Filter die Transmission von Licht kürzerer wellenlänge als des von dem ersten optischen Filter transmittierten Lichts zulässt.

5. Untersuchungsvorrichtung (1) nach Anspruch 4, wobei das Kennzeichnungsmaterial ein fluoreszierendes oder phosphoreszierendes Material oder ein Vorläufer davon ist und das erste optische Filter die Transmission von Licht nur nach der Modifikation durch das Kennzeichnungsmaterial zulassen wird.

6. Untersuchungsvorrichtung (1) nach Anspruch 1, wobei das erste optische Filter die Transmission von Licht verschiedener Wellenlängen zulässt, die Licht entsprechen, das von verschiedenen Kennzeichnungsmaterialien emittiert oder modifiziert wird.

7. Untersuchungsvorrichtung (1) nach Anspruch 1, die mehrere Bahnen (20) umfasst, die sich im Allgemeinen parallel zueinander von einer Aufbringungszone (14) zu einer Abfallzone (12) erstrecken, wobei die Bahnen (110) verschiedene Einfangmaterialien enthalten, um zu ermöglichen, dass ein Test an mehreren verschiedenen Analyten durchgeführt wird

8. Anordnung zur Durchführung einer Untersuchung, die aufweist: eine Untersuchungsvorrichtung (1) nach Anspruch 1 und ein Lesegerät (2) zum Erfassen einer Ausgabe der Untersuchungsvorrichtung (1), wobei das Lesegerät (2) aufweist: einen Körper mit einer Mündung (35), der fähig ist, die Untersuchungsvorrichtung (1) aufzunehmen, eine Lichtquelle (32) zum Beleuchten der Untersuchungsvorrichtung (1) von ihrer zweiten Seite, wenn sie einmal in dem Lesegerät (2) aufgenommen wurde, einen Anschluss zum Aufnehmen einer Leistungsquelle (36) für die Lichtquelle (32), einen optischen Detektor (34), der sich in dem Körper zum Detektieren von Licht, das von der ersten Seite der Untersuchungsvorrichtung emittiert wurde, befindet, und einen Detektor (38, 42) zum Delektieren der Lichtleistung von Licht, das von dem Lichtdetektor (34) detektiert wird.

9. Verfahren zum Durchführen eines Tests an einem Analyten mittels einer eigenständigen Untersuchungsvorrichtung (1) mit einer Aufbringungszone (14) für das Aufbringen des Analyten, einer Abfallzone (12) und einer Bahn (10), um den Durchgang des Analyten von der Aufbringungszone (14) zu der Abfallzone (12) zuzulassen, wobei die Untersuchungsvorrichtung (1) eine erste Abdeckung (6) auf einer ersten Seite der Bahn (10) und eine zweite Abdeckung (8) auf einer zweiten Seite der Bahn (10) gegenüber der ersten Seite hat, wobei die Bahn (10) auch eine Einfangzone (18) umfasst, die zwischen der Aufbringungszone (14) und der Abfallzone (12) angeordnet ist, die eine Menge an Einfangmaterial hat, das an die Bahn (10) gebunden ist, und die sich an einen Analysen binden wird, wobei das Verfahren aufweist:
Aufbringen einer Menge des Analyten auf die Aufbringzone (14) und Zulassen, dass der Analyt entlang der Bahn (10) zu einer Einfangzone (18), wo die Bahn (10) eine Menge an Einfangmaterial umfasst, das den Analyten an die Bahn (10) binden wird, und darüber hinaus läuft;
Bewirken, dass der Analyt entweder, bevor oder nachdem er die Einfangzone (18) erreicht hat, eine Menge eines Markierungsmaterials berührt, das wenigstens wenn es aktiviert wird, Licht emittieren oder modifizieren wird und das den Analyten binden wird, so dass der Analyt und das Kennzeichnungsmaterial in der Einfangzone (18) an die Bahn (10) gebunden sein werden;
Aufbringen einer Menge an Spülung auf die Aufbringzone (14), um zu bewirken, dass überschüssiger Analyt und Kennzeichnungsmaterial entlang der Bahn (10) zu der Abfallzone (12) fließen, und
Beleuchten der Untersuchungsvorrichtung (1) von der zweiten Seite der Bahn und Detektieren des Lichts, das von dem Kennzeichnungsmaterial emittiert oder modifiziert wird, von der ersten Seite der Bahn, wobei die erste Abdeckung (6) der Untersuchungsvorrichtung (1) ein erstes optisches Filter bereitstellt, um die Transmission von Licht zuzulassen, das von dem Kennzeichnungsmaterial emittiert oder modifiziert wird, und um Licht wenigstens eines anderen Wellenlängenbereichs zu sperren, so dass die Detektion des Lichts das Vorhandensein oder die Menge des Analyten anzeigen wird, und wobei die zweite Abdeckung der Untersuchungsvorrichtung (1) ein zweites optisches Filter bildet, das eine andere optische Transmissionscharakteristik als die des ersten optischen Filters hat.

10. Verfahren nach Anspruch 9, wobei jede der ersten und zweiten Abdeckungen (6, 8) ein einziges Stück Kunststoffmaterial aufweiset

11. Verfahren nach Anspruch 9, wobei das erste optische Filter und das zweite optische Filter zusammen im Wesentlichen jegliches Licht sperren

12. Verfahren nach Anspruch 9, das das Einsetzen der Untersuchungsvorrichtung (1) in ein Lesegerät (2) zum Detektieren einer Ausgabe von der Untersuchungsvorrichtung (1) aufweist, wobei das Lesegerät (2) einen Körper mit einer Mündung (35), der fähig ist, die Untersuchungsvorrichtung (1) aufzunehmen, eine Lichtquelle (32) zum Beleuchten der Untersuchungsvorrichtung (1) von ihrer zweiten Seite, wenn sie einmal in dem Lesegerät (2) aufgenommen wurde, einen Lichtdetektor (34), der sich in dem Körper befindet, um Licht, das von der ersten Seite der Untersuchungsvorrichtung (1) emittiert wurde, zu detektieren, und eine Anordnung (38, 42) zum Detektieren der Lichtleistung von Licht, das von dem Lichtdetektor (34) detektiert wird, aufweist.

13. Verfahren nach Anspruch 9, das das Beleuchten der Untersuchungsvorrichtung (1) mit weißem Licht von ihrer zweiten Seite und das Beobachten von ihrer ersten Seite aufweist, um das Vorhandensein oder die Menge des Analyten aus dem Kennzeichnungsmaterial zu bestimmen.

14. Verfahren nach Anspruch 13, wobei das weiße Licht Umgehungslicht ist

15. Verfahren nach Anspruch 13 oder Anspruch 14, wobei das Kennzeichnungsmaterial fluoreszierendes oder phosphoreszierendes Material aufweist.

## Revendications

1. Dispositif d'analyse autonome (1) destiné à permettre la mise en oeuvre d'un test sur une substance à analyser, le dispositif d'analyse (1) présentant une zone d'application (14) pour l'application d'une quantité de la substance à analyser, une zone de déchets (12), et un passage (10) pour permettre le passage de la substance à analyser, de la zone d'application (14) à la zone de déchets (12) ;
le dispositif d'analyse (1) présentant un premier cache (6) sur un premier côté du passage (10) et un second cache (8) sur un second côté du passage (10) opposé au premier côté ;
le premier cache (6) fournissant un premier filtre optique pour permettre la transmission de la lumière émise ou modifiée par un matériau indicateur, et pour bloquer la lumière d'au moins une autre plage de longueur d'onde ; et
le second cache (8) formant un second filtre optique, le second filtre optique présentant une caractéristique de transmission optique différente de celle du premier filtre optique ;
le dispositif d'analyse (1) incluant une quantité du matériau indicateurqui émettra ou modifiera la lumière, au moins lorsqu'il est activé, et qui se liera à la substance à analyser, le passage (10) incluant également une zone de capture (18), située entre la zone d'application (14) et la zone de déchets (12), laquelle présente une quantité de matériau de capture qui est lié au passage (10) et qui se liera à la substance à analyser qui transite le long du passage (10), de sorte que la substance à analyser sera liée au passage (10), le dispositif d'analyse (1) étant apte à être éclairé, à partir dudit second côté du passage, par une source lumineuse (32) séparée du dispositif d'analyse (1), de sorte que la lumière émise ou modifiée par le matériau indicateursitué dans le passage (10) est détectable au niveau du premier côté du passage.

2. Dispositif d'analyse (1) selon la revendication 1, dans lequel chacun des premier et second caches (6, 8) comporte une seule pièce en matière plastique.

3. Dispositif d'analyse (1) selon la revendication 1, dans lequel le premier filtre optique et le second filtre optique bloquent ensemble sensiblement toute la lumière.

4. Dispositif d'analyse (1) selon la revendication 1, dans lequel le second filtre optique permettra l'émission d'une lumière dont la longueur d'onde est plus courte que la lumière transmisepar le premier filtre optique.

5. Dispositif d'analyse (1) selon la revendication 4, dans lequel le matériau indicateur est un matériau fluorescent ou phosphorescent ou un précurseur connexe, et le premier filtre optique permettra la transmissionde la lumière uniquement suite à une modification par le matériau indicateur.

6. Dispositif d'analyse (1) selon la revendication 1, dans lequel le premier filtre optique permettra la transmissiond'une lumière de différentes longueurs d'onde correspondant à la lumière émise ou modifiée par différents matériaux indicateurs.

7. Dispositif d'analyse (1) selon la revendication 1, lequel inclut une pluralité de passages (10) s'étendant généralement parallèlement les uns aux autres, d'une zone d'application (14) à une zone de déchets (12), les passages (10) contenant différents matériaux de capture pour permettre la mise en oeuvre d'un test sur une pluralité de substances à analyser différentes.

8. Agencement destiné à mettre en oeuvre uneanalyse, lequel comprend un dispositif d'analyse (1) selon la revendication 1, et un lecteur (2) destiné à détecter une sortie en provenance du dispositif d'analyse (1), ledit lecteur (2) comprenant un corps présentant un orifice (35) qui est apte à recevoir le dispositif d'analyse (1), une source lumineuse (32) destinée à éclairer le dispositif d'analyse (1) à partir dudit second côté de celui-ci dès lors que le dispositif d'analyse a été reçu dans le lecteur (2), une borne destinée à recevoir une source d'alimentation (36) pour la source lumineuse (32), un détecteur optique (34) situé dans le corps en vue de détecter la lumière qui a été émise à partir du premier côté du dispositif d'analyse, et un détecteur (38, 42) destiné à détecter la puissance optique de la lumière détectée par le détecteur optique (34).

9. Procédé de mise en oeuvre d'un test sur une substance à analyser, au moyen d'un dispositif d'analyse autonome (1) présentant une zone d'application (14) pour l'application de la substance à analyser, une zone de déchets (12), et un passage (10) pour permettre le passage de la substance à analyser, de la zone d'application (14) à la zone de déchets (12), le dispositif d'analyse (1) présentant un premier cache (6) sur un premier côté du passage (10) et un second cache (8) sur un second côté du passage (10) opposé au premier côté, le passage (10) incluant également une zone de capture (18), située entre la zone d'application (14) et la zone de déchets (12), laquelle présente une quantité de matériau de capture qui est liée au passage (10) et qui se liera à une quelconque substance à analyser, le procédé comportant les étapes ci-dessous consistant à :
appliquer une quantité de la substance à analyser à la zone d'application (14) et permettre le passage de la substance à analyser le long du passage (10) vers et au-delà d'une zone de capture (18) lorsque le passage (10) inclut une quantité de matériau de capture qui liera la substance à analyser au passage (10) ;
amener la substance à analyser à entrer en contact, soit avant, soit après qu'elle a atteint la zone de capture (18), avec une quantité d'un matériau indicateur qui émettra ou modifiera la lumière, au moins lorsqu'il est activé, et qui se liera à la substance à analyser de sorte que la substance à analyser et le matériau indicateur seront liés au passage (10) au niveau de la zone de capture (18) ;
appliquer une quantité d'unecoucheà la zone d'application (14) afin d'amener un excès de substance à analyser ou de matériau indicateur à s'écouler le long du passage (10) vers la zone de déchets (12) ; et
éclairer le dispositif d'analyse (1) à partir du second côté du passage, et détecter la lumière qui est émise ou modifiée par le matériau indicateur à partir du premier côté du passage ;
ledit premier cache (6) du dispositif d'analyse (1) fournissant un premier filtre optique destiné à permettre la transmission de la lumière émise ou modifiée par le matériau indicateur, et à bloquer la lumière d'au moins une autre plage de longueur d'onde, de sorte que la détection de la lumière indiquera la présence ou la quantité de la substance à analyser, et ledit second cache du dispositif d'analyse (1) formant un second filtre optique présentant une caractéristique de transmission optique différente de celle du premier filtre optique.

10. Procédé selon la revendication 9, dans lequel chacun des premier et second caches (6, 8) comprend une seule pièce en matière plastique.

11. Procédé selon la revendication 9, dans lequel le premier filtre optique et le second filtre optique bloquent ensemble sensiblement toute la lumière.

12. Procédé selon la revendication 9, lequel comprend l'insertion du dispositif d'analyse (1) dans un lecteur (2) en vue de détecter une sortie en provenance du dispositif d'analyse (1), le lecteur (2) comprenant un corps présentant un orifice (35) qui est apte à recevoir le dispositif d'analyse (1), une source lumineuse (32) destinée à éclairer le dispositif d'analyse (1) à partir dudit second côté de celui-ci dès lors qu'il a été reçu dans le lecteur (2), un détecteur optique (34) situé dans le corps en vue de détecter la lumière qui a été émise à partir du premier côté du dispositif d'analyse (1), et un agencement (38, 42) destiné à détecter la puissance optique de la lumière détectée par le détecteur optique (34).

13. Procédé selon la revendication 9, lequel comprend une étape consistant à éclairer le dispositif d'analyse (1) au moyen d'une lumière blanche en provenance dudit second côté de celui-ci, et à observer à partir du premier côté de celui-ci en vue de déterminer la présence ou la quantité d'une substance à analyser à partir du matériau indicateur.

14. Procédé selon la revendication 13, dans lequel la lumière blanche est la lumière ambiante.

15. Procédé selon la revendication 13 ou 14, dans lequel le matériau indicateur comprend un matériau fluorescent ou phosphorescent.
